# EUROPEAN PATENT SPECIFICATION

(11) **EP 2 991 681 B1**
(45) Date of publication and mention of the grant of the patent: **12.06.2024**
(21) Application number: 14792127.4
(22) Date of filing: 02.05.2014
(51) Int. Cl.: A61K 9/00, A61K 8/34, A61K 47/10, A61K 8/365, A61K 8/368, A61K 8/42, A61K 8/49, A61Q 19/00, A61K 47/24, A61K 31/137, A61K 31/192, A61K 31/196, A61K 31/197, A61K 31/455, A61K 31/573, A61K 31/575, A61K 31/60, A61K 31/7056, A61K 8/55, A61K 31/167

(54) **TOPICAL COMPOSITION AND CARRIER FOR ADMINISTRATION OF PHARMACEUTICAL OR COSMETIC ACTIVE INGREDIENTS**
TOPISCHE ZUSAMMENSETZUNG UND TRÄGER ZUR VERABREICHUNG VON PHARMAZEUTISCHEN ODER KOSMETISCHEN WIRKSTOFFEN
COMPOSITION TOPIQUE ET VÉHICULE POUR L'ADMINISTRATION DE PRINCIPES ACTIFS PHARMACEUTIQUES OU COSMÉTIQUES

(30) Priority: 03.05.2013 SE 1300318
(43) Date of publication of application: 09.03.2016
(73) Proprietor: Emollivet AB, 41110 Göteborg (SE)
(72) Inventor: HERSLÖF, Bengt, S-114 21 Stockholm (SE); HOLMBÄCK, Jan, S-185 94 Vaxholm (SE)
(74) Representative: AWA Sweden AB
(86) International application number: PCT/SE2014/050546
(87) International publication number: WO 2014/178789

(56) References cited:
- WO-A1-01/60336
- WO-A1-2009/101412
- WO-A1-2011/056116
- WO-A1-2012/150890
- WO-A1-2012/150892
- WO-A1-2015/072910
- WO-A2-2010/036947
- CN-A- 101 829 052
- DE-C1- 4 021 084
- JP-A- 2008 163 010
- US-A1- 2002 076 423
- US-A1- 2003 170 194
- US-A1- 2006 147 383

## Description

### Field of the invention

The present invention relates to topical pharmaceutical or cosmetic compositions comprising a pharmaceutical or cosmetic carrier, to methods for their manufacture and to the use of said composition.

### Background of the invention

In the pharmaceutical and cosmetic field of topical formulations there is a need of a carrier capable of incorporating a pharmacologically or cosmetically active agent. Furthermore there is a need of a corresponding topical composition for administration comprising pharmacologically or cosmetically active agent. It is desirable that the composition can be applied onto the skin so as to form a thin coherent layer. To facilitate application, such as by spraying, the composition should be of low viscosity. The composition should furthermore facilitate the deposition of pharmacologically or cosmetically active agents to the skin. The composition should also have an acceptable shelf life.

WO 2010/036947 A2 discloses a lipid based pharmaceutical composition for topical administration comprising one or more lipids and one or more pharmaceutically active compounds selected from the group consisting of finasteride, duasteride, minoxidil, amphotericin B and tacrolimus.

EP 1 787 658 A1 discloses a sustained release formulation for subcutaneous or intramuscular administration, comprising somatostatin analogue inhibitor of growth hormone, C₁-C₈ alcohol, phospholipid, and C₁-C₄ alkyl fatty acid ester.

JP 2008-163010 discloses a non-aqueous stock solution containing phosphatide (phospholipid) and C₂-C₅ aliphatic alcohol, combined with a propellant which contains 50 weight % or more of dimethyl ether. The stock solution can contain 0.005 to 4 weight % of phosphatide.

US 2002/0076423 relates to cooling cosmetic or dermatological formulations which reduce the secondary reactions of the skin to the effect of UV radiation, containing chitosan and lecithin. Compositions exemplified are emulsions containing at least 25 % of water. DE 40 21 084 C1 describes a topical composition comprising 1-10% phospholipid, 0,1-20% alcohol, 1-5% urea and a large excess of water in aqueous solution.

### Disclosure of the invention

It is an object of the invention to provide a carrier and a composition, which is easily applicable onto the skin, such as by spraying.

Still an object of the invention is to provide a carrier capable of incorporating a pharmacologically and cosmetically active agent.

Still an object of the invention is to provide a composition capable of forming a stable coherent layer on the skin of an animal, such as a mammal, including humans.

Still an object of the invention is to provide a composition in which the components are physically and chemically stable during an acceptable shelf life. Further objects of the invention will be evident from examples.

The present invention is directed to a sprayable topical pharmaceutical or cosmetic composition comprising a pharmaceutical or cosmetic carrier comprising from 1 % by weight to 55 % by weight of a phospholipid; a C2 -C4 alcohol selected from the group consisting of ethanol, n-propanol, isopropanol, n-butanol, isobutanol and t-butanol; a keratolytic agent selected from the group consisting of glycolic acid, lactic acid, malic acid, salicylic acid, allantoin, urea and sulphur; up to 2 % by weight of water; and a pharmacologically or cosmetically active agent dissolved in said carrier, which composition is a clear liquid, and wherein said composition is sprayable by a pump device.

An aspect of the present invention is a topical pharmaceutical or cosmetic composition comprising a carrier as disclosed herein, and a pharmacologically or cosmetically active agent dissolved therein. The topical pharmaceutical or cosmetic composition of the invention can be formed by a method comprising:(a) providing said carrier; (b) admixing a pharmaceutically or cosmetically active agent; (c) agitating said mixture obtained in step (b), optionally under heating, until a clear liquid has been formed.

The present invention is based on the insight that lower alcohol, in particular a C₂-C₄ alcohol, can be advantageously used as the evaporating component, in particular the single evaporating component, of a composition for topical administration comprising a phospholipid, a keratolytic agent and a pharmacologically or cosmetically active agent. Preferred lower alcohol includes ethanol, n-propanol, isopropanol, n-butanol, isobutanol and t-butanol.

Phospholipids of the invention include, but not limited to, commercial pharmaceutical or cosmetic grade phospholipid, and can be natural or synthetic. A person skilled in the art is aware of that phospholipid from commercial sources comprises substantial amounts of other lipids. Phospholipid useful in accordance with the invention may contain substantial amounts of non-polar lipids. It may be composed of up to about 50 % by weight of mono-, di- and triglycerides. Natural phospholipids include, but are not limited to, lecithins from soybeans, rape seeds, sunflower seeds and eggs. A preferred phospholipid of the invention is phosphatidylcholine (PC), a major constituent of cell membranes.

Keratolytic agents of the invention include α- and β-hydroxy acids, selected from the group consisting of glycolic acid, lactic acid, malic acid, and salicylic acid and pharmaceutically acceptable salts thereof. Preferred keratolytic agents of the invention are allantoin, urea and sulphur. A particularly preferred keratolytic agent is urea. The carriers and compositions of the invention are clear colorless, light yellow or brownish yellow fluids that can be stored for long periods of time, even at elevated temperatures, such as 30 °C or 40 °C, without change in physical appearance, such as precipitation, cloudiness or phase separation.

The clear appearance and low viscosity of the carrier and composition of the invention seems to be due to the inability of phospholipids to form lyotropic liquid crystals, such as lamellar and hexagonal of high viscosity in the solvent system used. The carrier and the pharmaceutical or cosmetic composition of the invention are clear and of low viscosity even at concentrations of phospholipid as high as 50-60 % by weight. In contrast, phospholipid compositions corresponding to those of the invention, but which contain substantial amounts of water, are slightly viscous dispersions at low polar lipid concentrations or thick gels at higher polar lipid concentrations. The high viscosity of the latter composition does not allow administration by spraying.

The low viscosity of the carrier and composition of the invention makes them suitable for spraying by a pump device instead of using propellants and pressurized containers.

The pharmacologically active agent of the invention is selected from the group consisting of: antimicrobial agent, antibiotic; antimycotic agent; antibacterial agent; antifungal agent; antiviral agent; antiseptic; anti-phlogistic; anti-pruritic agent; anti-psoriatic agent; antitussive agent; anti-alopecia agent; anti-acne agent; anti-inflammatory agent; antiphlogistics; analgesic; antiulcer agent; local anaesthetic; immune response modifying agent.

More particularly, the pharmacologically active agent of the invention is selected from: antibacterial agents, such as oxytetracycline, fusidic acid, gentamycine, mupirocin, retapamulin (and pharmaceutically acceptable salts and derivatives thereof); antimycotic agents, such as nystatin, clotrimazole, miconazole, econazole, ketoconazole, bifonazole, and combinations of imidazole and triazole derivatives, ciclopirox, terbinafine, fluconazole, and amorolfine (and pharmaceutically acceptable salts and derivatives thereof); antiviral agents, such as aciclovir, valaciclovir, penciclovir, famciclovir, foscarnet (sodium phosphoneformate hexahydrate) and docosanol (and pharmaceutically acceptable salts and derivatives thereof); antiseptics, such as chlorhexidine, benzalkonium chloride and hydrogen peroxide; anti-inflammatory agents (glucocorticoids), such as hydrocortisone, clobetasone, triamcinolone, betamethasone, mometasone, desonide, prednisolone and clobetasol (and pharmaceutically acceptable salts and derivatives thereof); antiphlogistics/analgesics (NSAID's), such as acetylsalicylic acid, diclofenac, ketoprofen, ibuprofen, naproxen, capsaicin and nicotinate (and pharmaceutically acceptable salts and derivatives thereof); antipruritic agents, such as glucocorticoids, for example, hydrocortisone, clobetasone, clobetasol, desonide, mometasone and betamethasone, and local anaesthetics, for example, lidocaine, prilocaine, ropivacaine, mepivacaine, bupivacaine, levobupivacaine, benzocaine, and tetracaine (and pharmaceutically acceptable salts and derivatives thereof); antipsoriatic agents, such as calcipotriol, calcitriol, 7-dehydrocholesterol, cholecalciferol, maxacalcitol, doxercalciferol, paricalcitol, inecalcitol, eldecalcitol, tacalcitol, betamethasone and cyclosporine A (and pharmaceutically acceptable salts and derivatives thereof); agents for treatment of eczema and atopic dermatitis: tacrolimus and pimecrolimus (and pharmaceutically acceptable salts and derivatives thereof); antiglaucomateous agents, such as timolol, betaxolol, latanoprost, bimatoprost, and travoprost (and pharmaceutically acceptable salts and derivatives thereof); agents for erectile dysfunction, such as alprostadil (prostaglandin E1) (and pharmaceutically acceptable salts and derivatives thereof); anti-dandruff agents, such as selenium sulphides, piroctone oleamine and ketoconazole; anti-alopecia agents, such as minoxidil (and pharmaceutically acceptable salts and derivatives thereof); anti-acne agents, such as retinol, tretinoin (retinoic acid), isotretinoin, adapalene, motretinide, benzoyl peroxide, clindamycin azelaic acid and lauric acid (and pharmaceutically acceptable salts and derivatives thereof); wound healing agents, such as pantothenic acid and fusidic acid (and pharmaceutically acceptable salts and derivatives thereof); steroid hormones, such as prednisone, dexamethasone, estradiol, triamcinolone, fludrocortisone, testosterone, distilbestrol; peptide homones, such as oxytocin, LL-37, DPK-060 and PXL-01 (and pharmaceutically acceptable salts and derivatives thereof).

The cosmetically active agent of the invention is preferably selected from the group consisting of: antiperspirant; antisudoral agent; antidandruff agent; glidant and moisturizing agent.

According to one aspect of the invention the pharmaceutical or cosmetic carrier comprises or substantially consists of 30 % by weight to 75 % by weight of a phospholipid, from 20 % by weight to 60 % by weight of a C₂ to C₄ alcohol, from 0.05 % by weight to 10 % by weight of a keratolytic agent, and optionally 1-2 % by weight of water, adding up to 100 %.

According to another aspect of the invention the pharmaceutical or cosmetic carrier comprises or substantially consists of 30 % by weight to 65 % by weight of a phospholipid, from 30 % by weight to 60 % by weight of a C₂ to C₄ alcohol, from 0.05 % by weight to 10 % by weight of a keratolytic agent and optionally 1-2 % by weight of water, adding up to 100 %.

According to another aspect of the invention the weight ratio of phospholipid to C₂ to C₄ alcohol of the carrier is from 1.5:1 to 1:1.5 or from 1.2:1 to 1:1.2, such as about 1:1, the carrier consisting of 90 % by weight or more of phospholipid and C₂ to C₄ alcohol in combination, of 10 % by weight or less of a keratolytic agent and, optionally, of up to 1 % by weight or up to 2 % by weight of water, the combined contents adding up to 100 %.

According to another aspect of the invention the pharmaceutical or cosmetic composition of the invention comprises or substantially consists of 30 % by weight to 75 % by weight of phospholipid, from 20 % by weight to 60 % by weight of C₂ to C₄ alcohol, from 0.05 % by weight to 10 % by weight of a keratolytic agent, from 0.001 % by weight to 5 % by weight, exceptionally up to 8 % by weight, of pharmacologically or cosmetically active agent, and optionally 1-2 % by weight of water, adding up to 100 %.

According to another aspect of the invention the pharmaceutical or cosmetic composition comprises or substantially consists of 40 % by weight to 65 % by weight of a phospholipid, from 30 % by weight to 60 % by weight of a C₂ to C₄ alcohol, from 0.05 % by weight to 10 % by weight of a keratolytic agent, from 0.001 % by weight to 5 % by weight, exceptionally up to 8 % by weight, of a pharmacologically or cosmetically active agent and optionally 1-2 % by weight of water, adding up to 100 %.

According to another aspect of the invention the weight ratio of phospholipid to C₂ to C₄ alcohol of the pharmaceutical or cosmetic composition is from 1:1.5 to 1.5:1 or from 1:1.2 to 1.2:1, such as about 1:1, the composition consisting of 85 % by weight or more of phospholipid and C₂ to C₄ alcohol in combination, of 10 % by weight or less of keratolytic agent, of up to 5 % by weight and, exceptionally, up to 8 % by weight of pharmacologically or cosmetically active agent and, optionally, of up to 1 % by weight or up to 2 % by weight of water, the combined components adding up to 100 %.

According to an aspect of the invention, there is provided a topical pharmaceutical or cosmetic composition comprising a pharmaceutical or cosmetic carrier comprising
at least 3 % by weight of a phospholipid;
at least 20 % by weight of a C₂-C₄ alcohol;
at least 0.05 % by weight of a keratolytic agent; and
optionally, comprising up to 2 % by weight of water; and pharmacologically or cosmetically active agent dissolved in said carrier.

In one embodiment of the invention, said composition comprises from 3 % by weight to 60 % by weight of a phospholipid; from 20 % by weight to 90 % by weight of a C₂-C₄ alcohol; from 0.05 % by weight to 15 % by weight of a keratolytic agent; from 0.001 % by weight to 8 % by weight of pharmacologically or cosmetically active agent; optionally further comprising water up to 2 % by weight; wherein the components are added up to a total of 100% by weight.

In another embodiment of the invention, said composition comprises from 5 % by weight to 55 % by weight of a phospholipid, from 30 % by weight to 85 % by weight of a C₂-C₄ alcohol; from 0.05 % by weight to 10 % by weight of a keratolytic agent; from 0.001 % by weight to 8 % by weight of a pharmacologically or cosmetically active agent; optionally further comprising water up to 2 % by weight; wherein the components are added up to a total of 100% by weight.

In another embodiment of the invention, said composition comprises 5-20 % by weight of a phospholipid.

In another embodiment of tthe invention, said composition comprises 10-20 % by weight of a phospholipid.

In another embodiment of the invention, said composition comprises about 5, 6, 7, 8, 9 or 10 % by weight of a phospholipid.

In another embodiment ofthe invention, said composition comprises 70-90 % by weight of a C₂-C₄ alcohol.

In another embodiment of the invention, said composition comprises 0.5-8 % by weight of a keratolytic agent.

The C₂-C₄ alcohol is selected from the group consisting of ethanol, n-propanol, isopropanol, n-butanol, isobutanol and t-butanol.

In another embodiment of the invention, said C₂-C₄ alcohol is ethanol.

In another embodiment of the invention, said phospholipid comprises or substantially consists of phosphatidylcholine (PC).

In another embodiment of the invention, said keratolytic agent is selected from the group consisting of glycolic acid; lactic acid; malic acid; salicylic acid; allantoin; urea and sulphur.

In another embodiment of the invention, said keratolytic agent is urea.

In another embodiment of the invention, said pharmacologically active agent is selected from antimicrobial agent; antibiotic; antimycotic agent; antibacterial agent; antifungal agent; antiviral agent; antiseptic; anti-phlogistic; anti-pruritic agent; anti-psoriatic agent; antitussive agent; anti-alopecia agent; anti-acne agent; anti-inflammatory agent; antiphlogistics; analgesic; antiulcer agent; local anaesthetic and immune response modifying agent.

In another embodiment of the invention, said pharmacologically active agent is a peptide.

In another embodiment of this aspect, said cosmetically active agent is selected from an antiperspirant; an antisudoral agent; an antidandruff agent; a glidant and a moisturizing agent.

In another embodiment of the invention, said composition is in sprayable form.

According to an aspect of the invention, there is provided a topical pharmaceutical or cosmetic carrier comprising a phospholipid, a C₂-C₄ alcohol, and a keratolytic agent, the carrier comprising of
at least 5 % by weight of a phospholipid;
at least 20 % by weight of a C₂-C₄ alcohol;
at least 0.05 % by weight of a keratolytic agent; and
optionally, comprising up to 2 % by weight of water.

In one embodiment of the invention, said carrier comprises from 5 % by weight to 60 % by weight of a phospholipid; from 20 % by weight to 90 % by weight of a C₂-C₄ alcohol; and from 0.05 % by weight to 15 % by weight of a keratolytic agent.

In another embodiment of the invention, said carrier comprises from 10 % by weight to 55 % by weight of a phospholipid, from 30 % by weight to 85 % by weight of a C₂-C₄ alcohol; and from 0.05 % by weight to 10 % by weight of a keratolytic agent.

In another embodiment of the invention, said carrier comprises from about 5, 6, 7, 8, 9 or 10 % by weight of a phospholipid.

In another embodiment of the invention, said carrier comprises 5-20 % by weight of a phospholipid.

In another embodiment of the invention, said carrier comprises 10-20 % by weight of a phospholipid.

In another embodiment of the invention, said carrier comprises 70-90 % by weight of a C₂-C₄ alcohol.

In another embodiment of the invention, said carrier comprises 0.5-8 % by weight of a keratolytic agent.

The C₂-C₄ alcohol is selected from the group consisting of ethanol, n-propanol, isopropanol, n-butanol, isobutanol and t-butanol.

In another embodiment of the invention, said C₂-C₄ alcohol is ethanol.

In another embodiment of the invention, said phospholipid comprises or substantially consists of phosphatidylcholine (PC).

In another embodiment of the invention, said keratolytic agent is selected from the group consisting of glycolic acid; lactic acid; malic acid; salicylic acid; allantoin; urea and sulphur.

In another embodiment of the invention, said keratolytic agent is urea.

In another embodiment of the invention, said carrier is stable for at least 3 months of storage, at room temperature.

According to an aspect of the invention, there is provided a method of preparing a topical pharmaceutical or cosmetic composition according to the present invention, comprising:
(a) providing carrier according to the present invention;
(b) admixing a pharmaceutically or cosmetically active agent;
(c) agitating said mixture obtained in step (b), optionally under heating, until a clear liquid has been formed.

According to an aspect of the invention, there is provided the use of a topical pharmaceutical composition according to the present invention, for administration of a pharmacologically active agent contained therein. Said administration may be by spraying.

According to an aspect of the invention, there is provided the use of a topical cosmetic composition according to the present invention, for administration of an active agent contained therein. Said administration may be by spraying.

According to an aspect of the invention, there is provided veterinary use of a topical pharmaceutical or cosmetic composition according to the present invention, for administration of an active agent contained therein. Said administration may be by spraying.

According to an aspect of the invention, there is provided a method of treating a disease in a patient or animal in need thereof comprising topically administering a pharmaceutical composition according to the present invention, said composition comprising a therapeutically active amount of a pharmacologically active ingredient. Said administration may be topically administered by spraying.

The carrier of the invention is particularly suited for incorporation of pharmacologically active peptides such as protease inhibitors, insulin, growth hormone, interferons, interleukins, pentagetide, histamine releasing peptide antigen, antiflammins, corticotropin releasing factor, interferon-γ antagonists, somatostatin, calcium channel peptide, opiate agonists such as E-2078 and dynorphin A, opiate antagonists, sleep inducing peptide,
calcitonin, PTH-releasing peptide, growth hormone releasing peptide, LHRH agonists such as buserelin, goserelin, leuprolide, LHRH antagonists, anticoagulants such as hirudin and hirudin analogs, desmopressin and desmopressin analogs, melanoma receptor blockers, captopril, oxytocin, vasopressin. Urea is a particularly preferred keratolytic agent in combination with peptides by promoting dissolution of peptide in the carrier.

According to an aspect of the invention there is provided a sprayable topical pharmaceutical or cosmetic composition comprising from 1 % by weight to 55 % or 60 % by weight, in particular from 20 % by weight to 55 % by weight, and from 35 % by weight or 40 % by weight to 50 % by weight or 55 % by weight or more of a phospholipid. The sprayable composition comprises additionally a C₂ to C₄ alcohol, a keratolytic agent, and a pharmaceutically or cosmetically active agent.

The sprayable pharmaceutical or cosmetic composition preferably consists of from 20 % by weight to 55 % by weight of a phospholipid, from 40 % by weight to 75 % by weight of a C₂ to C₄ alcohol, from 0.05 % by weight to 8 % by weight of a keratolytic agent, and from 0.001 % by weight to 6 % by weight, more preferred from 0.05 % by weight to 4 % by weight, of a pharmaceutically or cosmetically active agent, the combined components adding up to 100 %. In the sprayable composition of the invention the upper limit of sprayability is primarily controlled by phospholipid content. While compositions with a phospholipid content of up to 55 % by weight are sprayable those with a phospholipid content of 60 % usually are not. The upper limit of sprayability may however also be influenced by the content of keratolytic agent and pharmacologically or cosmetically active agent, in particular if their combined content exceeds 3 % by weight, so as to lower the upper limit of sprayability somewhat.

In one embodiment of the invention, said sprayable topical pharmaceutical or cosmetic composition comprises
at least 5 % by weight of a phospholipid;
at least 20 % by weight of a C₂-C₄ alcohol;
at least 0.05 % by weight a keratolytic agent; and
optionally, comprising up to 2 % by weight of water.

In one embodiment of the invention, said sprayable topical pharmaceutical or cosmetic composition comprises from 5 % by weight to 60 % by weight of a phospholipid; from 20 % by weight to 90 % by weight of a C₂-C₄ alcohol; and from 0.05 % by weight to 15 % by weight of a keratolytic agent.

In one embodiment of the invention, said sprayable topical pharmaceutical or cosmetic composition comprises from 10 % by weight to 55 % by weight of a phospholipid, from 30 % by weight to 85 % by weight of a C₂-C₄ alcohol; and from 0.05 % by weight to 10 % by weight of a keratolytic agent

In one embodiment of the invention, said sprayable topical pharmaceutical or cosmetic composition comprises from about 5, 6, 7, 8, 9 or 10 % by weight of a phospholipid.

In one embodiment of the invention, said sprayable topical pharmaceutical or cosmetic composition comprises 5-20 % by weight of a phospholipid.

In one embodiment of the invention, said sprayable topical pharmaceutical or cosmetic composition comprises 10-20 % by weight of a phospholipid.

In one embodiment of the invention, said sprayable topical pharmaceutical or cosmetic composition comprises 70-90 % by weight of a C₂-C₄ alcohol.

In one embodiment of the invention, said sprayable topical pharmaceutical or cosmetic composition comprises 0.5-8 % by weight of a keratolytic agent.

In one embodiment of the invention, said sprayable topical pharmaceutical or cosmetic composition comprises ethanol as a C₂-C₄ alcohol.

In one embodiment of the invention, said phospholipid comprises or substantially consists of phosphatidylcholine (PC).

In one embodiment of the invention, said keratolytic agent is selected from the group consisting of glycolic acid; lactic acid; malic acid; salicylic acid; allantoin; urea and sulphur.

In one embodiment of the invention, said keratolytic agent is urea.

In one embodiment of the invention, there is provided a carrier of the invention, which is stable for at least 3 months of storage, at room temperature.

According to an one aspect of the invention, the solubility of compounds of widely differing HLB-values (hydrophilic/lipophilic-balance) or LogP values (logarithm of octanol/water partition coefficient) in PC/ethanol mixtures increases in parallel with the concentration of phospholipid. This feature is shared by the carrier of the invention and the composition of the invention.

According to one aspect of the invention, the carrier and the composition of the invention is suitable for topical treatment of human, animal or mammalian animal skin due to rapid evaporation of the alcohol.

According to one aspect of the invention, the properties of the continuous film or layer on the skin can be varied by incorporating other lipids or solvents, for example isopropylmyristate, mono-, di- and triglycerides, silicone oils or propylene glycol.

The pharmaceutical or cosmetic composition of the invention can be prepared by providing carrier of the invention; admixing pharmaceutically or cosmetically active agent to obtain a mixture; agitating the mixture, optionally under heating, until a clear liquid has been formed.

The composition of the invention can be used for topical administration, in particular by spraying, of pharmacologically or cosmetically active agent contained therein.

The pharmaceutical or cosmetic composition of the invention may be used on humans and other animals, such as mammals. Consequently, veterinary use of the compositions of the invention is included.

### Materials used in the examples

**Table 1. Lipids used in the Examples**

| **Trade name** | **Chemical name** | **Supplier** | **CAS No.** |
|---|---|---|---|
| Lipoid S75 | Soybean lecithin | Lipoid | 8002-43-5 |
| Lipoid S100 | Soybean lecithin | Lipoid | 8002-43-5 |
| Phospholipon 90G | Soybean lecithin | Lipoid | 8002-43-5 |
| Capmul MCM C8 EP | Medium chain monoglycerides, Glycerol monocaprylate | Abitec Corp. | 26402-26-6 |
| Isopropyl myristate (IPM) | Isopropyl myristate | Aldrich | 110-27-0 |

Alcohols used in the examples were ethanol 99.9% ("EtOH", VWR), 2-propanol (isopropanol, HPLC grade, Rathburn), and 2-butanol (ReagentPlus^{®}, Sigma-Aldrich).The silicone oil used in the examples was Cyclomethicone 5-NF (Dow Corning, decamethylcyclopentasiloxane). Peptide LL-37 was from PolyPeptide Laboratories A/S Peptide DPK-060 was from Dermagen AB and Peptide PXL-01 was from Pergamum AB. All other substances were from Sigma-Aldrich.

### EXAMPLE 1

### Carriers of the invention

The carriers of the invention listed in Tables 2-4 were prepared. The phospholipid was dissolved in the alcohol to the desired concentration. If necessary, the dissolution was promoted by short ultrasonication at 25-40 °C in a water bath sonicator. A pre-weighed amount of keratolytic agent was added and the mixture gently heated and ultrasonicated until a clear liquid had been formed.

**Table 2. Carriers of the invention (% by weight)**

| **Carrier #** | **Phospholipid** | **%** | **Alcohol** | **%** | **Keratolyic agent** | **%** |
|---|---|---|---|---|---|---|
| 2a | Lipoid 5100 | 3.0 | Ethanol | 95.0 | Urea | 2.0 |
| 2b | Lipoid 5100 | 5.0 | Ethanol | 94.0 | Urea | 1.0 |
| 2c | Lipoid 5100 | 5.0 | Ethanol | 93.0 | Urea | 2.0 |
| 2d | Lipoid 5100 | 10.0 | Ethanol | 88.0 | Urea | 2.0 |
| 2e | Lipoid S100 | 20.0 | Ethanol | 78.0 | Urea | 2.0 |
| 2f | Lipoid S100 | 30.0 | Ethanol | 68.0 | Urea | 2.0 |
| 2g | Lipoid S100 | 19.8 | Ethanol | 73.1 | Urea | 5.0 |
| | | | | | Glycolic acid | 2.1 |
| 2h | Lipoid S100 | 20.0 | Ethanol | 75.0 | Urea | 5.0 |
| 2i | Lipoid S100 | 20.2 | Ethanol | 74.9 | Lactic acid | 4.9 |
| 2j | Lipoid S100 | 20.2 | Ethanol | 74.9 | Salicylic acid | 4.9 |
| 2k | Lipoid S75 | 4.0 | 2-propanol | 89.0 | Urea | 2.0 |
| | | | | | Lactic acid | 5.0 |

**Table 3. Carriers of the invention with a high phospholipid content (% by weight)**

| **Carrier #** | **Phospholipid** | **%** | **Alcohol** | **%** | **Keratolytic agent** | **%** |
|---|---|---|---|---|---|---|
| 3a | Lipoid 5100 | 48.0 | Ethanol | 48.0 | Urea | 4.0 |
| 3b | Lipoid S75 | 48.0 | Ethanol | 48.0 | Urea | 4.0 |
| 3c | Lipoid S100 | 48.0 | 2-Propanol | 48.0 | Urea | 4.0 |
| 3d | Lipoid S100 | 47.0 | Ethanol | 47.0 | Lactic acid | 6.0 |
| 3e | Lipoid S100 | 49.5 | Ethanol | 49.5 | Sodium lactate | 1.0 |
| 3f | Lipoid S100 | 47.5 | Ethanol | 47.5 | Glycolic acid | 5.0 |
| 3g | Lipoid S100 | 48.5 | 2-Butanol | 49.3 | Salicylic acid | 2.2 |
| 3h | Lipoid S75 | 30.1 | Ethanol | 68.1 | Allantoin | 0.05 |
| | | | | | Water | 1.7 |
| 3i | Lipoid S75 | 48.6 | Ethanol | 46.0 | Salicylic acid | 5.4 |
| 3j | Lipoid S100 | 49.5 | Ethanol | 49.5 | Urea | 1.0 |

**Table 4. Carriers of the invention with additional lipid or solvent (% by weight)**

| **Carrier #** | **Phospholipid** | **%** | **Alcohol** | **%** | **Keratolytic agent** | **%** | **Additional component** | **%** |
|---|---|---|---|---|---|---|---|---|
| 4:1 | Lipoid S-100 | 24.5 | Ethanol | 47.3 | Urea | 4.7 | MCM | 23.5 |
| 4:2 | Lipoid S-100 | 9.5 | Ethanol | 77.7 | Urea | 3.3 | MCM/IPM 1:1 | 9.4 |
| 4:3 | Phospholipon 90G | 10.0 | Ethanol | 74.9 | Urea | 5.0 | MCM/IPM 1:1 | 10.0 |
| 4:4 | Lipoid S-75 | 29.8 | Ethanol | 45.8 | Urea | 3.5 | MCM/IPM 1:1 | 20.9 |
| 4:5 | Lipoid S-100 | 29.7 | Ethanol | 45.9 | Urea | 4.2 | MCM/IPM 1:1 | 20.3 |
| 4:6 | Lipoid S-100 | 30.0 | 2-Propanol | 10.0 | Urea | 4.6 | MCM/IPM 1:1 | 20.1 |
| | | | Ethanol | 35.3 | | | | |
| 4:7 | Lipoid S-100 | 9.6 | Ethanol | 79.4 | Lactic acid | 1.5 | MCM/IPM 1:1 | 9.5 |
| 4:8 | Lipoid S-100 | 25.1 | Ethanol | 39.1 | Lactic acid | 6.0 | MCM/IPM 1:1 | 29.8 |
| 4:9 | Lipoid S-100 | 30.5 | Ethanol | 48.7 | Sodium lactate | 1.1 | MCM/IPM 1:1 | 19.7 |
| 4:10 | Lipoid S-100 | 28.9 | Ethanol | 43.5 | Glycolic acid | 4.8 | MCM/IPM 1:1 | 22.8 |
| 4:11 | Lipoid S-100 | 9.9 | Ethanol | 79.6 | Salicylic acid | 0.5 | MCM/IPM 1:1 | 10.0 |
| 4:12 | Lipoid S-100 | 29.5 | 2-Butanol | 10.0 | Salicylic acid | 2.1 | MCM/IPM 1:1 | 20.6 |
| | | | Ethanol | 37.9 | | | | |
| 4:13 | Lipoid S-100 | 9.5 | Ethanol | 76.1 | Urea | 3.2 | MCM/IPM 1:1 | 9.5 |
| | | | | | Lactic acid | 1.7 | | |
| 4:14 | Lipoid S-100 | 10.0 | Ethanol | 73.2 | Urea | 4.8 | MCM/IPM 1:1 | 10.0 |
| | | | | | Glycolic acid | 2.1 | | |
| 4:15 | Lipoid S-100 | 14.9 | Ethanol | 73.8 | Urea | 5.1 | MCM/IPM 1:1 | 5.1 |
| | | | | | Glycolic acid | 1.0 | | |
| 4:16 | Lipoid S-100 | 3.0 | Ethanol | 82.0 | Urea | 5.0 | Propylene glycol | 10.0 |
| 4:17 | Lipoid S-100 | 4.8 | Ethanol | 69.9 | Urea | 4.8 | Propylene glycol | 20.4 |
| 4:18 | Phospholipon 90G | 5.1 | Ethanol | 74.8 | Urea | 5.0 | Propylene glycol | 15.2 |
| 4:19 | Lipoid S-100 | 9.6 | Ethanol | 65.6 | Urea | 5.0 | Propylene glycol | 19.8 |
| 4:20 | Lipoid S-100 | 18.7 | Ethanol | 57.0 | Urea | 5.0 | Propylene glycol | 19.4 |
| 4:21 | Lipoid S-100 | 20.0 | Ethanol | 75.0 | Urea | 2.5 | Propylene glycol | 2.5 |
| 4:22 | Lipoid S-100 | 20.4 | Ethanol | 74.7 | Urea | 2.5 | Propylene glycol | 2.4 |
| 4:23 | Lipoid S-100 | 11.2 | Ethanol | 27.2 | Salicylic acid | 1.6 | Cyclomethicone 5-NF | 50.6 |
| | | | | | | | MCM/IPM 1:1 | 9.5 |

### EXAMPLE 2

### Composition of the invention

Examples of the composition of the invention are listed in Tables 5, 6 and 7. They were prepared by adding a pre-weighed amount of the respective cosmetic or pharmacologically active agent to one of the carriers of Example 1. The mixtures were gently heated and ultrasonicated until clear liquids had been formed.

**Table 5. Compositions of the invention**

| **Composition #** | **Carrier #** | **% By weight** | **Active agent** | **% By weight** |
|---|---|---|---|---|
| 5:1 | 2a | 95.3 | Diclofenac sodium | 4.7 |
| 5:2 | 2b | 95.3 | Ibuprofen | 4.7 |
| 5:3 | 2c | 98.0 | Ketoprofen | 2.0 |
| 5:4 | 2d | 97.8 | Naproxen | 2.2 |
| 5:5 | 2e | 99.6 | Cyclosporin A | 0.4 |
| 5:6 | | 98.5 | Calcium pantothenate | 1.5 |
| 5:7 | 2f | 99.0 | Capsaicin | 1.0 |
| 5:8 | 2g | 98.8 | Retinol | 1.2 |
| 5:9 | 2h | 99.0 | Clindamycin hydrochloride | 1.0 |
| 5:10 | | 91,9 | Lauric acid | 8.1 |
| 5:11 | | 98.0 | Sodium fusidate | 2.0 |
| 5:12 | | 99.7 | Curcumin | 0.3 |
| 5:13 | | 99.1 | Tacrolimus | 0.9 |
| 5:14 | | 99.9 | Mometasone furcate | 0.10 |
| 5:15 | 2i | 98.5 | Diclofenac Sodium | 1.5 |
| 5:16 | 2j | 99.6 | Naproxen | 0.4 |
| 5:17 | 2k | 99.6 | Ketoprofen | 0.4 |

**Table 6. Compositions of the invention with a high phospholipid content (% by weight)**

| **Composition #** | **Carrier #** | **% By weight** | **Active agent** | **% By weight** |
|---|---|---|---|---|
| 6:1 | 3a | 99.9 | Betamethasone dipropionate | 0.1 |
| 6:2 | | 99.995 | Calcipotriol | 0.005 |
| 6:3 | | 95.6 | Diclofenac sodium | 4.4 |
| 6:4 | | 99.72 | Mupirocin | 0.28 |
| 6:5 | | 98.0 | Peptide LL-37 | 2.0 |
| 6:6 | 3b | 99.4 | Benzalkonium chloride | 0.6 |
| 6:7 | | 99.0 | Benzoyl peroxide | 1.0 |
| 6:8 | | 99.8 | Betamethasone valerate | 0.2 |
| 6:9 | | 99.1 | Chlorhexidine | 0.9 |
| 6:10 | | 98.2 | Econazole nitrate | 1.8 |
| 6:11 | | 98.7 | Hydrocortisone | 1.3 |
| 6:12 | | 99.1 | Hydrocortisone butyrate | 0.9 |
| 6:13 | | 98.7 | Peptide DPK-060 | 1.3 |
| 6:14 | | 99.5 | Peptide PXL-01 | 0.5 |
| 6:15 | | 99.0 | Oxytocin acetate | 1.0 |
| 6:16 | 3c | 98.4 | Hydrocortisone | 1.6 |
| 6:17 | | 98.0 | Diclofenac sodium | 2.0 |
| 6:18 | | 98.9 | Oxytocin acetate | 1.1 |
| 6:19 | 3d | 99.0 | Estradiol | 1.0 |
| 6:20 | | 97.2 | Diclofenac sodium | 2.8 |
| 6:21 | | 99.0 | Peptide DPK-060 | 1.0 |
| 6:22 | | 98.7 | Peptide LL-37 | 1.3 |
| 6:23 | 3e | 96.3 | Diclofenac sodium | 3.7 |
| 6:24 | | 98.9 | Estradiol | 1.1 |
| 6:25 | 3f | 98.8 | Estradiol | 1.2 |
| 6:26 | | 99.4 | Peptide PXL-01 | 0.6 |
| 6:27 | 3g | 99.0 | Estradiol | 1.0 |
| 6:28 | 3h | 99.0 | Diclofenac sodium | 1.0 |

**Table 7. Compositions of the invention with additional lipid or solvent (% by weight)**

| **Composition #** | **Carrier #** | **% By weight** | **Active agent** | **% By weight** |
|---|---|---|---|---|
| 7:1 | 4:1 | 99.0 | Diclofenac sodium | 1.0 |
| 7:2 | 4:2 | 99.7 | Curcumin | 0.3 |
| 7:3 | 4:3 | 99.0 | Hydrocortisone | 1.0 |
| 7:4 | 4:7 | 99.7 | Curcumin | 0.3 |
| 7:5 | 4:12 | 92.0 | Lauric acid | 8.0 |
| 7:6 | 4:13 | 99.7 | Curcumin | 0.3 |
| 7:7 | 4:23 | 99.1 | Terbinafine hydrochloride | 0.99 |

### EXAMPLE 3

### Comparative test 1 - anesthesia

A composition of the invention comprising anaesthetic agent was compared in respect of onset of action of active agent with a corresponding composition lacking keratolytic agent (Table 8).

**Table 8. Comparative test in respect of onset of action of active agent**

| **Component** | **Composition A % by weight** | **Composition B* % by weight** |
|---|---|---|
| Phospholipid (Lipoid S75) | 41.4 | 43.7 |
| Absolute ethanol | 45.1 | 47.0 |
| Urea | 5.2 | 0 |
| Lidocaine (active agent) | 4.4 | 4.6 |
| Prilocaine (active agent) | 3.9 | 4.1 |

| | | |
|---|---|---|
| * Composition not comprised by the invention. | | |

The compositions were prepared by the method of Examples 1 and 2. The compositions A and B (10 µl each) were applied on the right and left volar forearms, respectively, of a male subject so as to cover skin areas of about 1 cm². Ten min after application a slight numbness was felt on the right forearm area but not on the left forearm area. This indicates a faster onset of action of composition A.

### EXAMPLE 4

### Comparative test 2 - vasodilation

Skin color measurements were used to study the onset time of methyl nicotinate induced erythema of different compositions according to methods known in the art (Bonina F P et al., In vitro and in vivo evaluation of polyoxyethylene esters as dermal prodrugs of ketoprofen, naproxen and diclofenac. Europ J Pharm Sci 14 (2001) 123-134; Duval C et al., Difference among moisturizers in affecting skin susceptibility to hexyl nicotinate, measured as time to increase skin blood flow. Skin Res Techn 9 (2003) 59-63; Wiren K et al., Enhancement of bioavailability by lowering of fat content in topical formulations. Br J Dermat 160 (2009) 552-556). Three formulations containing methyl nicotinate were applied to areas on the skin of both volar forearms of a male subject (age 56). Skin color was measured by using DSM II Colormeter (Cortex Technology, Denmark) which is based on an active color detecting chip where illumination is provided by white LEDs.The measured parameter (erythema index, E.I.) corresponds to the redness of the skin (Bonina F P et al., *supra*). The erythema effect (ΔE.I.) was calculated as the difference between the measured E.I and the baseline, and the onset time as the time needed to reach 75% of the maximum erythema effect after application. The figures shown in Table 9 are mean values from four treatments, after application of 6 µl of the compositions on circular test areas of 3 cm². The results show that adding keratolytic agents to a phospholipid containing composition shortens the onset time of methyl nicotinate induced erythema.

**Table 9. Onset time of methyl nicotinate induced erythema by various compositions**

| Composition # | Lipoid 5100 (% w/w) | Ethanol (% w/w) | Keratolytic agent | % w/w) | Methyl nicotinate (% w/w) | Onset time (min) |
|---|---|---|---|---|---|---|
| 9:1* | 25.3 | 74.3 | - | - | 0.4 | 9.9 |
| 9:2 | 25.2 | 68.9 | Urea | 5.5 | 0.4 | 8.3 |
| 9:3 | 25.1 | 69.5 | Glycolic acid | 5.0 | 0.4 | 8.9 |

| | | | | | | |
|---|---|---|---|---|---|---|
| * Composition not comprised by the invention | | | | | | |

### EXAMPLE 5

### Comparative test 3 - vasodilation

A composition of the invention comprising vasodilating agent was compared in respect of duration of action of active agent with a corresponding composition lacking keratolytic agent (Table 10).

**Table 10. Comparative test in respect of duration of action of active agent**

| **Component** | **Composition A of the invention % by weight** | **Composition B % by weight** |
|---|---|---|
| Phospholipid (Lipoid S100) | 47.5 | 47.5 |
| Absolute ethanol | 47.5 | 47.5 |
| Urea | 5.0 | 0 |
| Methyl nicotinate (active agent) | 0.1 | 0.1 |

The compositions were prepared by the method of Examples 1 and 2. The compositions A and B (10 µl each) were applied on the inside forearms of a male subject. Each application covered a skin area of about 1 cm². 15 min after application redness of the same intensities appeared on the application spots. After approximately 1 hour redness caused by composition B had faded while that caused by composition A had not. The longer duration of action of composition A indicates a better penetration into the skin than of composition B.

### EXAMPLE 6

### Comparative test 4 - tape stripping of curcumin treated skin

Skin color measurements were used to study the penetration of curcumin into the skin. Two formulations containing curcumin were applied to areas on the skin of the left volar forearm of a male subject (age 56). After application the test area was stripped with adhesive tape ten times. Skin color was measured using the same instrument as in Example 4. The measured quantity, the b* parameter in the CIE 1976 (L*, a*, b*) color space, was found to have a linear relationship with the amount of curcumin absorbed by the skin. The relative amount of curcumin in the skin was calculated as the difference between the measured b* and the baseline (Δb*) after ten tape strippings, divided by the difference measured immediately after application. The numbers shown in Table 11 are mean values from two treatments, after application of 5 µl of the compositions on circular test areas of 3 cm².

The results indicate that adding keratolytic agents to a phospholipid containing composition enhances the penetration of curcumin into the skin.

**Table 11. Relative amount remaining in the skin after application of various curcumin compositions and ten subsequent strippings by adhesive tape.**

| Composition # | Lipoid 5100 (% w/w) | Ethanol (% w/w) | IPM+MCM 1:1 (% w/w) | Urea (% w/w) | Curcumin (% w/w) | Rel. Amount curcumin after stripping |
|---|---|---|---|---|---|---|
| 11:1* | 9.5 | 80.7 | 9.4 | - | 0.3 | 0.36 |
| 11:2 | 9.5 | 77.5 | 9.4 | 3.3 | 0.3 | 0.46 |

| | | | | | | |
|---|---|---|---|---|---|---|
| * Composition not comprised by the invention | | | | | | |

### EXAMPLE 7

### Antifungal composition

By the method of Example 1, carrier of the invention was prepared from 39.5 parts by weight of phospholipid (Lipoid S75), 53.1 parts by weight of absolute ethanol and 6.5 parts by weight of urea. One part by weight of terbinafine hydrochloride (active agent) was added to the carrier and the mixture gently heated and ultrasonicated until a clear liquid had been formed.

### EXAMPLE 8

### Antiperspirant composition

By the method of Example 1 carrier of the invention was prepared from 47.9 parts by weight of phospholipid, 47.9 parts by weight of absolute ethanol and 3.9 parts by weight of urea. Aluminum chloride hexahydrate (0.3 parts by weight, active agent) was added to the carrier and the mixture gently heated and ultrasonicated until a clear liquid had been formed.

### EXAMPLE 9

### Increase of solubility of active agent in a carrier by raising the carrier phospholipid content

The dissolution capacity of different carriers was tested by admixing controlled amounts of active agent. The results are listed in Table 12. The results show that increasing concentration of phospholipids in the carrier increases the dissolution capacity for substances in a wide range of polarity.

**Table 12. Comparison of carrier dissolution capacity. Amount of active agent in % by weight**

| | | | Carrier (%w/w) | | | Tested composition (% w/w) | |
|---|---|---|---|---|---|---|---|
| | | | | | | Lowest incomplete dissolution | Highest complete dissolution |
| Tested substance | HLB* | LogP** | Lipoid S100 | Urea | Ethanol | | |
| Sucrose ester | 2 | 7-10 | 5.0 | 1.0 | 94.0 | 0.14 | |
| | | | 49.5 | 1.0 | 49.5 | | 0.18 |
| Sucrose ester | 6 | 4-7 | 5.0 | 1.0 | 94.0 | 0.50 | |
| | | | 49.5 | 1.0 | 49.5 | | 3.35 |
| | | | 0.0 | 2.0 | 98.0 | 0.65 | 0.59 |
| | | | 5.0 | 2.0 | 93.0 | 1.10 | 0.83 |
| | | | 10.0 | 2.0 | 88.0 | 0.98 | 0.87 |
| | | | 20.0 | 2.0 | 78.0 | 1.45 | 1.22 |
| Sucrose ester | 11 | 2-4 | 5.0 | 1.0 | 94.0 | 9.90 | |
| | | | 49.5 | 1.0 | 49.5 | | 16.83 |
| Hydrocortisone | | 1.6 | 5.0 | 1.0 | 94.0 | 2.28 | |
| | | | 49.5 | 1.0 | 49.5 | | 2.89 |
| | | | 0.0 | 2.0 | 98.0 | 2.25 | 2.09 |
| | | | 5.0 | 2.0 | 93.0 | 2.37 | 2.22 |
| | | | 10.0 | 2.0 | 88.0 | 2.83 | 2.48 |
| | | | 20.0 | 2.0 | 78.0 | 3.22 | 2.82 |
| | | | 30.0 | 2.0 | 68.0 | 3.50 | 3.19 |
| Calcium pantothenate | | -1.1 | 0.0 | 2.0 | 98.0 | 0.40 | 0.27 |
| | | | 3.4 | 2.0 | 94.7 | | 0.41 |
| | | | 5.0 | 2.0 | 93.0 | | 0.50 |
| | | | 10.0 | 2.0 | 88.0 | | 0.98 |
| | | | 20.0 | 2.0 | 78.0 | | 1.81 |
| | | | 30.0 | 2.0 | 68.0 | | 3.17 |

| | | | | | | | |
|---|---|---|---|---|---|---|---|
| *HLB: Hydrophilic-lipophilic balance **LogP (logarithm of octanol-water partition coefficient): Experimental value for hydrocortisone. Estimated value for calcium pantothenate. Rough estimates from theoretical calculations on average structures for sucrose esters. | | | | | | | |

### EXAMPLE 10

### Physical and chemical stability of carriers and compositions

The carriers 4:14 and 4:15 were analyzed for content of urea and degradation products after 4 months at 30 °C by HPLC and NMR spectroscopy. The measured remaining concentration of urea was 100 %, 97 % and 95 %, respectively, and no formation of degradation products could be detected in any of the compositions.

The topical pharmaceutical composition 7:7 and the antifungal composition from Example 7 were analyzed for content of terbinafine hydrochloride after approximately 15 months at room temperature by HPLC. The measured remaining content was 95 % and 100 %, respectively.

Composition 7:7 was also analyzed for content of salicylic acid after 15 months at 30 °C. The measured remaining content was 104 %.

The composition from Example 7 was analyzed for content of urea and degradation products after 15 months at room temperature by HPLC and NMR spectroscopy. No decrease in urea concentration and no formation of degradation products could be detected.

The topical pharmaceutical composition 6:3 was analyzed for content of diclofenac sodium by HPLC after 11 months at room temperature. No decrease in diclofenac sodium content could be detected

The results show that the carriers and compositions of the invention have a surprisingly good physical stability, as well as chemical stability for both the keratolytic agent and the incorporated active substance.

## Claims

1. A sprayable topical pharmaceutical or cosmetic composition comprising a pharmaceutical or cosmetic carrier comprising
from 1 % by weight to 55 % by weight of a phospholipid;
a C₂-C₄ alcohol selected from the group consisting of ethanol, n-propanol, isopropanol, n-butanol, isobutanol and t-butanol;
a keratolytic agent selected from the group consisting of glycolic acid, lactic acid, malic acid, salicylic acid, allantoin, urea and sulphur;
up to 2 % by weight of water; and
a pharmacologically or cosmetically active agent dissolved in said carrier, which composition is a clear liquid, and wherein said composition is sprayable by a pump device.

2. The composition of claim 1, comprising from 20 % by weight to 55 % by weight, such as from 35 % by weight to 55 % by weight, such as 40 % by weight to 55 % by weight of a phospholipid.

3. The composition according to claim 1 or 2, wherein the composition consists of from 20 % by weight to 55 % by weight of a phospholipid, from 40 % by weight to 75 % by weight of said C₂ to C₄ alcohol, from 0.05 % by weight to 8 % by weight of said keratolytic agent, and from 0.001 % by weight to 6 % by weight of a pharmacologically or cosmetically active agent, the combined components adding up to 100 %.

4. The composition of claim 1, wherein said keratolytic agent is urea.

5. The composition according to any one of claims 1 to 4, wherein said C₂ to C₄ alcohol is ethanol.

6. The composition according to any one of claims 1 to 5, wherein said pharmacologically active agent is selected from antimicrobial agent; antibiotic; antimycotic agent; antibacterial agent; antifungal agent; antiviral agent; antiseptic; anti-phlogistic; anti-pruritic agent; anti-psoriatic agent; antitussive agent; anti-alopecia agent; anti-acne agent; anti-inflammatory agent; antiulcer agent; local anaesthetic and immune response modifying agent.

7. The composition according to any one of claims 1 to 5, wherein said cosmetically active agent is selected from an antiperspirant; an antisudoral agent; an antidandruff agent; a glidant and a moisturizing agent.

8. The composition according to any one of claims 1-7, wherein the phospholipid is phosphatidylcholine.

9. A topical pharmaceutical or cosmetic carrier comprising a phospholipid, a C₂-C₄ alcohol, and a keratolytic agent, the carrier comprising:
from 1 % by weight to 55 % by weight of a phospholipid;
a C₂-C₄ alcohol alcohol selected from the group consisting of ethanol, n-propanol, isopropanol, n-butanol, isobutanol and t-butanol;
a keratolytic agent selected from the group consisting of glycolic acid; lactic acid; malic acid; salicylic acid; allantoin; urea and sulphur; and
up to 2 % by weight of water,
wherein said carrier is a clear liquid and wherein said carrier is sprayable by a pump device.

10. The carrier of claim 9, comprising from 20 % by weight to 55 % by weight, such as from 35 % by weight to 55 % by weight, such as 40 % by weight to 55 % by weight of a phospholipid.

11. The carrier according to any one of claims 9 to 10, wherein said keratolytic agent is urea.

12. The carrier according to any one of claims 9 to 11, wherein said C₂ to C₄ alcohol is ethanol.

13. A method of preparing a topical pharmaceutical or cosmetic composition according to any one of claims 1 to 8 comprising:
(a) providing carrier according to any one of claims 9 to 12;
(b) admixing a pharmaceutically or cosmetically active agent;
(c) agitating said mixture obtained in step (b), optionally under heating, until a clear liquid has been formed.

14. A topical pharmaceutical composition according to any one of claims 1 to 8 for use in treatment of psoriasis, wherein the the pharmacologically active agent is an anti-psoriatic agent.

15. A topical pharmaceutical composition according to any one of claims 1 to 8, or a carrier according to any one of claims 9 to 12, for use in treatment of eczema, wherein the pharmacologically acitve agent is an agent for treatment of eczema.

16. Use of a carrier according to any one of claims 9 to 12 for preparing a pharmaceutical composition suitable for topical administration.

17. Non-therapeutic use of a topical cosmetic composition according to any one of claims 1 to 8 or a topical cosmetic carrier according to any one of claims 9 to 12, for administration of a cosmetic agent.

## Patentansprüche

1. Versprühbare topische pharmazeutische oder kosmetische Zusammensetzung, umfassend einen pharmazeutischen oder kosmetischen Träger, umfassend 1 Gew.-% bis 55 Gew.-% eines Phospholipids,
einen C₂-C₄-Alkohol ausgewählt aus der Gruppe bestehend aus Ethanol, n-Propanol, Isopropanol, n-Butanol, Isobutanol und t-Butanol,
ein keratolytisches Mittel ausgewählt aus der Gruppe bestehend aus Glykolsäure, Milchsäure, Äpfelsäure, Salicylsäure, Allantoin, Harnstoff und Schwefel,
bis zu 2 Gew.-% Wasser und
einen pharmakologischen oder kosmetischen Wirkstoff gelöst in diesem Träger, wobei es sich bei der Zusammensetzung um eine klare Flüssigkeit handelt und wobei die Zusammensetzung mittels einer Pumpvorrichtung versprühbar ist.

2. Zusammensetzung nach Anspruch 1, umfassend 20 Gew.- % bis 55 Gew.-%, wie 35 Gew.-% bis 55 Gew.-%, wie 40 Gew.-% bis 55 Gew.-% eines Phospholipids.

3. Zusammensetzung nach Anspruch 1 oder 2, wobei die Zusammensetzung aus 20 Gew.-% bis 55 Gew.-% eines Phospholipids, 40 Gew.-% bis 75 Gew.-% des C₂- bis C₄-Alkohols, 0,05 Gew.-% bis 8 Gew.-% des keratolytischen Mittels und 0,001 Gew.-% bis 6 Gew.-% eines pharmakologischen oder kosmetischen Wirkstoffs besteht, wobei die Summe der Komponenten 100 % ergibt.

4. Zusammensetzung nach Anspruch 1, wobei es sich bei dem keratolytischen Mittel um Harnstoff handelt.

5. Zusammensetzung nach einem der Ansprüche 1 bis 4, wobei es sich bei dem C₂- bis C₄-Alkohol um Ethanol handelt.

6. Zusammensetzung nach einem der Ansprüche 1 to 5, wobei der pharmakologische Wirkstoff aus einem antimikrobiellen Mittel, einem Antibiotikum, einem Antimykotikum, einem antibakteriellen Mittel, einem Antipilzmittel, einem antiviralen Mittel, einem Antiseptikum, einem Antiphlogistikum, einem Antipruritikum, einem Antipsoriatikum, einem Antitussivum, einem Mittel gegen Haarausfall, einem Mittel gegen Akne, einem entzündungshemmenden Mittel, einem Mittel gegen Magengeschwüre, einem Lokalanästhetikum und einem Mittel zum Modifizieren der Immunantwort ausgewählt ist.

7. Zusammensetzung nach einem der Ansprüche 1 bis 5, wobei der kosmetische Wirkstoff aus einem schweißhemmenden Mittel, einem Antisudorikum, einem Antischuppenmittel, einem Gleitmittel und einem Feuchthaltemittel ausgewählt ist.

8. Zusammensetzung nach einem der Ansprüche 1-7, wobei es sich bei dem Phospholipid um Phosphatidylcholin handelt.

9. Topischer pharmazeutischer oder kosmetischer Träger, umfassend ein Phospholipid, einen C₂-C₄-Alkohol und ein keratolytisches Mittel, wobei der Träger Folgendes umfasst:
1 Gew.-% bis 55 Gew.-% eines Phospholipids,
einen C₂-C₄-Alkohol ausgewählt aus der Gruppe bestehend aus Ethanol, n-Propanol, Isopropanol, n-Butanol, Isobutanol und t-Butanol,
ein keratolytisches Mittel ausgewählt aus der Gruppe bestehend aus Glykolsäure, Milchsäure, Äpfelsäure, Salicylsäure, Allantoin, Harnstoff und Schwefel und
bis zu 2 Gew.-% Wasser,
wobei es sich bei dem Träger um eine klare Flüssigkeit handelt und wobei der Träger mittels einer Pumpvorrichtung versprühbar ist.

10. Träger nach Anspruch 9, umfassend 20 Gew.-% bis 55 Gew.-%, wie 35 Gew.-% bis 55 Gew.-%, wie 40 Gew.-% bis 55 Gew.-% eines Phospholipids.

11. Träger nach Anspruch 9 oder 10, wobei es sich bei dem keratolytischen Mittel um Harnstoff handelt.

12. Träger nach einem der Ansprüche 9 bis 11, wobei es sich bei dem C₂- bis C₄-Alkohol um Ethanol handelt.

13. Verfahren zur Herstellung einer topischen pharmazeutischen oder kosmetischen Zusammensetzung nach einem der Ansprüche 1 bis 8, bei dem man:
(a) einen Träger nach einem der Ansprüche 9 bis 12 bereitstellt,
(b) mit einem pharmazeutischen oder kosmetischen Wirkstoff mischt,
(c) die in Schritt (b) erhaltene Mischung rührt, gegebenenfalls unter Erhitzen, bis sich eine klare Flüssigkeit gebildet hat.

14. Topische pharmazeutische Zusammensetzung nach einem der Ansprüche 1 bis 8 zur Verwendung bei der Behandlung von Psoriasis, wobei es sich bei dem pharmakologischen Wirkstoff um ein Antipsoriatikum handelt.

15. Topische pharmazeutische Zusammensetzung nach einem der Ansprüche 1 bis 8 oder Träger nach einem der Ansprüche 9 bis 12 zur Verwendung bei der Behandlung von Ekzemen, wobei es sich bei dem pharmakologischen Wirkstoff um ein Mittel zur Behandlung von Ekzemen handelt.

16. Verwendung eines Trägers nach einem der Ansprüche 9 bis 12 zur Herstellung einer für die topische Verabreichung geeigneten pharmazeutischen Zusammensetzung.

17. Nichttherapeutische Verwendung einer topischen kosmetischen Zusammensetzung nach einem der Ansprüche 1 bis 8 oder eines topischen kosmetischen Trägers nach einem der Ansprüche 9 bis 12 zur Verabreichung eines kosmetischen Mittels.

## Revendications

1. Composition pharmaceutique ou cosmétique topique pulvérisable comprenant un support pharmaceutique ou cosmétique comprenant
de 1 % en poids à 55 % en poids d'un phospholipide ;
un alcool en C₂-C₄ sélectionné dans le groupe constitué par l'éthanol, le n-propanol, l'isopropanol, le n-butanol, l'isobutanol et le t-butanol ;
un agent kératiolytique sélectionné dans le groupe constitué par l'acide glycolique, l'acide lactique, l'acide malique, l'acide salicylique, l'allantoïne, l'urée et le soufre ;
jusqu'à 2 % en poids d'eau ; et
un agent pharmacologiquement ou cosmétiquement actif dissous dans ledit support, laquelle composition étant un liquide limpide, et dans laquelle ladite composition est pulvérisable par un dispositif à pompe.

2. Composition selon la revendication 1, comprenant de 20 % en poids à 55 % en poids, par exemple de 35 % en poids à 55 % en poids, par exemple 40 % en poids à 55 % en poids d'un phospholipide.

3. Composition selon la revendication 1 ou 2, dans laquelle la composition est constituée de 20 % en poids à 55 % en poids d'un phospholipide, de 40 % en poids à 75 % en poids dudit alcool en C₂-C₄, de 0,05 % en poids à 8 % en poids dudit agent kératiolytique, et de 0,001 % en poids à 6 % en poids d'un agent pharmacologiquement ou cosmétiquement actif, les composants combinés totalisant 100 %.

4. Composition selon la revendication 1, dans laquelle ledit agent kératiolytique est l'urée.

5. Composition selon l'une quelconque des revendications 1 à 4, dans laquelle ledit alcool en C₂-C₄ est l'éthanol.

6. Composition selon l'une quelconque des revendications 1 à 5, dans laquelle ledit agent pharmacologiquement actif est sélectionné parmi un agent antimicrobien ; un antibiotique ; un agent antimycotique ; un agent antibactérien ; un agent antifongique ; un agent antiviral ; un antiseptique ; un anti-phlogistique ; un agent anti-prurigineux ; un agent anti-rhumatisme psoriasique ; un agent antitussif ; un agent anti-alopécie ; un agent anti-acné ; un agent anti-inflammatoire ; un agent anti-ulcère ; un anesthésique local et agent de modification de la réponse immunitaire.

7. Composition selon l'une quelconque des revendications 1 à 5, dans laquelle ledit agent cosmétiquement actif est sélectionné parmi un antitranspirant ; un agent anti-sudoral ; un agent antipelliculaire ; un agent glissant et un agent hydratant.

8. Composition selon l'une quelconque des revendications 1-7, dans laquelle le phospholipide est la phosphatidylcholine.

9. Support cosmétique ou pharmaceutique topique comprenant un phospholipide, un alcool en C₂-C₄ et un agent kératiolytique, le support comprenant :
de 1 % en poids à 55 % en poids d'un phospholipide ;
un alcool en C₂-C₄ sélectionné dans le groupe constitué par l'éthanol, le n-propanol, l'isopropanol, le n-butanol, l'isobutanol et le t-butanol ;
un agent kératiolytique sélectionné dans le groupe constitué par l'acide glycolique ; l'acide lactique ; l'acide malique ; l'acide salicylique ; l'allantoïne ; l'urée et le soufre ; et
jusqu'à 2 % en poids d'eau,
dans lequel ledit support est un liquide limpide et dans lequel ledit support est pulvérisable par un dispositif à pompe.

10. Support selon la revendication 9, comprenant de 20 % en poids à 55 % en poids, par exemple de 35 % en poids à 55 % en poids, par exemple 40 % en poids à 55 % en poids d'un phospholipide.

11. Support selon l'une quelconque des revendications 9 à 10, dans lequel ledit agent kératiolytique est l'urée.

12. Support selon l'une quelconque des revendications 9 à 11, dans lequel ledit alcool en C₂-C₄ est l'éthanol.

13. Procédé de préparation d'une composition pharmaceutique ou cosmétique topique selon l'une quelconque des revendications 1 à 8, comprenant :
(a) la fourniture du support selon l'une quelconque des revendications 9 à 12 ;
(b) le mélange d'un agent pharmaceutiquement ou cosmétiquement actif ;
(c) l'agitation dudit mélange obtenu à l'étape (b), éventuellement en chauffant, jusqu'à ce qu'un liquide limpide soit formé.

14. Composition pharmaceutique topique selon l'une quelconque des revendications 1 à 8 pour une utilisation dans le traitement du psoriasis, dans laquelle l'agent pharmacologiquement actif est un agent anti-psoriasique.

15. Composition pharmaceutique topique selon l'une quelconque des revendications 1 à 8, ou support selon l'une quelconque des revendications 9 à 12, pour utilisation dans le traitement de l'eczéma, dans laquelle l'agent pharmacologiquement actif est un agent de traitement de l'eczéma.

16. Utilisation d'un support selon l'une quelconque des revendications 9 à 12 pour la préparation d'une composition pharmaceutique appropriée pour une administration topique.

17. Utilisation non thérapeutique d'une composition cosmétique topique selon l'une quelconque des revendications 1 à 8 ou d'un support cosmétique topique selon l'une quelconque des revendications 9 à 12, pour l'administration d'un agent cosmétique.
